(19) **Europäisches Patentamt**

**European Patent Office**

**Office européen des brevets**

(11) Veröffentlichungsnummer: **0 028 295**
**B1**

---

(12) # EUROPÄISCHE PATENTSCHRIFT

(45) Veröffentlichungstag der Patentschrift:
**24.03.82**

(21) Anmeldenummer: **80104866.1**

(22) Anmeldetag: **16.08.80**

(51) Int. Cl.³: **C 07 C 27/16,** C 07 C 51/34,
C 07 C 178/00, C 07 B 3/00

---

(54) **Verfahren zur Umsetzung von Olefinen in carbonsaurem Medium mit Ozon.**

---

(30) Priorität: **19.10.79 DE 2942279**

(43) Veröffentlichungstag der Anmeldung:
**13.05.81 Patentblatt 81/19**

(45) Bekanntmachung des Hinweises auf die Patenterteilung:
**24.03.82 Patentblatt 82/12**

(84) Benannte Vertragsstaaten:
**BE FR GB IT NL**

(56) Entgegenhaltungen:
**EP 80 104 866.1**
**DE-A1-2 713 863**
**DE-B-1 283 822**
**US-A-3 748 262**

(73) Patentinhaber: **CHEMISCHE WERKE HÜLS AG,**
**Postfach 1320, D-4370 Marl 1 (DE)**

(72) Erfinder: **Dohm, Klaus Dieter, Dr.,**
**Friedrich-Hebbel-Strasse 3, D-4358 Haltern (DE)**
Erfinder: **Hofmann, Peter, Dr., Lipper Weg 193,**
**D-4370 Marl (DE)**

---

## Verfahren zur Umsetzung von Olefinen in carbonsaurem Medium mit Ozon

Die Umsetzung von olefinischen Substraten mit Ozon (Ozonolyse) ist eine seit langem bekannte Reaktion, die über ihre präparative und analytische Bedeutung hinaus in zunehmendem Masse in der chemischen Industrie als Syntheseverfahren Eingang findet. Als Einsatzolefine sind dabei sowohl lineare als auch cyclische Kohlenwasserstoffe mit einer oder mehreren Doppelbindungen geeignet. Aus ökonomischen Gründen ist der Einsatz relativ teuren Ozons vor allem bei höheren Olefinen mit nur einem relativ geringen spezifischen Ozonverbrauch pro Masseneinheit des Olefins und einem hohen Veredlungsgrad des oder der Endprodukte interessant.

Je nach Verfahrensweise gelangt man durch Ozonolyse zu peroxidischen, aldehydischen und/oder carbonsauren Folgeprodukten bzw. deren Derivaten [P. S. Bailey, Chem. Rev. 58, 925 (1958)].

In der Technik bleibt man in der Regel nicht auf der Stufe der peroxidischen Ozonolyseprodukte stehen, sondern unterwirft diese im allgemeinen einer Nachbehandlung, um so zu stabilen Reaktionsprodukten zu gelangen. Da Ozonide und di- bzw. oligomere Peroxide meist nicht einfach und nur mit mässiger Ausbeute in stabile Endprodukte überführbar sind, führt man dann, wenn eine weitere Umsetzung der Ozonolyseprodukte beabsichtigt ist, die Ozonolysereaktion in sogenannten teilnehmenden Lösungsmitteln, wie Alkoholen und Carbonsäuren, durch. Ein besonders bevorzugtes Lösungmittel sind hierbei Carbonsäuren, die im Gegensatz zu Alkoholen von Ozon nicht oxidativ angegriffen werden. Durch geeignete Weiterverarbeitung in solchen Lösungsmitteln erhaltener Reaktionsprodukte — wie Reduktion, Thermolyse oder oxidative Thermolyse — gelangt man zu Aldehyden, Aldehyd-Carbonsäure-Gemischen oder zu Carbonsäuren. Beim Einsatz cyclischer Olefine gelangt man entsprechend zu Dialdehyden, Aldehydcarbonsäuren oder Dicarbonsäuren.

Als Einsatzgas für die Ozonerzeugung kommen neben Luft reiner Sauerstoff und sauerstoffhaltige Gasmischungen in Frage. Jedoch selbst beim Einsatz des gegenüber Luft und sauerstoffhaltigen Gasmischungen mit wirtschaftlichen Vorteilen verbundenen reinen Sauerstoffs bleiben 90 Volumenprozent und mehr des für die Ozonerzeugung eingesetzten Gases ungenutzt. Daraus ergibt sich für relativ teure Einsatzgase, wie Sauerstoff, sauerstoffreiche Gasmischungen und mit Sauerstoff angereicherter Luft, das Problem der wirtschaftlich sinnvollen Nutzung des nach der Ozonolysereaktion verbleibenden praktisch ozonfreien Restgases. Nur in seltenen Fällen wird es möglich sein, das bei der Ozonolyse anfallende Restgas ohne weitere Reinigung für eine andere Produktion einzusetzen. Selbst wenn dies der Fall ist, hat man alle Nachteile zweier miteinander gekoppelter Prozesse. Der daher vorzugsweise einzuschlagende Weg für eine Nutzung des Restgases ist eine Rückführung nach entsprechender Reinigung in die Ozonerzeugung.

Die bereits bekannte Gasreinigung durch elektrostatische Abscheidung gemäss dem Verfahren der US-PS 2 813 113 stellt noch keine allgemeine befriedigende Lösung dar. Die hohen Spannungen, mit denen derartige Anlagen betrieben werden, können nämlich durch elektrischen Überschlag zur Funkenbildung und damit zur Zündung des mit organischer Substanz beladenen sauerstoffhaltigen Gases führen. Ausserdem beeinträchtigt die mit dem Gas in die Reinigungsanlage eingebrachte Feuchtigkeit zusätzlich die Betriebssicherheit derartiger elektrostatischer Abscheider.

Aufgabe der vorliegenden Erfindung war es daher, ein Verfahren zu entwickeln, das es gestattet, den bei der Umsetzung von Olefinen mit Ozon in carbonsaurem Medium die Ozonolysestufe verlassenden Gasstrom (Sauerstoff oder sauerstoffhaltige Gasmischungen) in möglichst betriebssicherer und wirtschaftlicher Weise so zu reinigen, dass er dem Ozongenerator wieder zugeführt werden kann.

Diese Aufgabe wurde durch die in den Ansprüchen beschriebenen Massnahmen gelöst.

Als Olefine können bei dem erfindungsgemässen Verfahren sowohl lineare als auch cyclische einfach oder mehrfach ungesättigte Kohlenwasserstoffe eingesetzt werden. Aus Sicherheitsgründen sollte jedoch auf den Einsatz von Olefinen mit weniger als 5 Kohlenstoffatomen verzichtet werden. Im allgemeinen verwendet man Olefine mit 6 bis 30, vorzugsweise 8 bis 24 Kohlenstoffatomen. Typische Vertreter aus der Reihe der linearen und cyclischen Olefine sind z.B. α-Olefine mit C-Zahlen von 12 bis 18, Ölsäure, Elaidinsäure, Erucasäure, Cycloocten, Cyclododecen, Cyclooctadien und Cyclododecatrien.

Geeignete Carbonsäuren sind solche mit bis zu 12 Kohlenstoffatomen. Bevorzugt werden jedoch Monocarbonsäuren mit bis zu 4 Kohlenstoffatomen, wie z.B. Ameisensäure, Essigsäure und Propionsäure, eingesetzt. Aufgrund ihres verglichen mit Ameisensäure geringeren korrosiven Charakters, ihrer wohlfeilen Verfügbarkeit, ihres günstigen Lösevermögens und Siedepunkts ist dabei der Essigsäure und der Propionsäure der Vorzug zu geben. Neben den Carbonsäuren können, sofern es für die Durchführung des Verfahrens zweckmässig ist, auch Carbonsäureanhydride eingesetzt werden .

Die Carbonsäure(n) wird bzw. werden im allgemeinen mindestens in molaren Mengen, bezogen auf das Olefin, eingesetzt. Bei Verwendung olefinischer Ausgangsmaterialien, die bereits eine oder mehrere Carboxylgruppen tragen, ist allerdings auch der Einsatz geringerer als molarer Carbonsäure möglich (in etwa bis herab zu 0,5 Mol Carbonsäure pro Mol Olefin). Als zweckmässig hat es sich jedoch erwiesen, pro Gewichtseinheit Olefin 2 bis 20 Gewichtseinheiten Carbonsäure zu verwenden.

Als Einsatzgase für die Ozonerzeugung kommen Luft, sauerstoffhaltige Gasmischungen mit z.B. Stickstoff, Argon und Kohlendioxid, die wenigstens 20 Volumenprozent Sauerstoff enthalten, und reiner Sauerstoff in Frage.

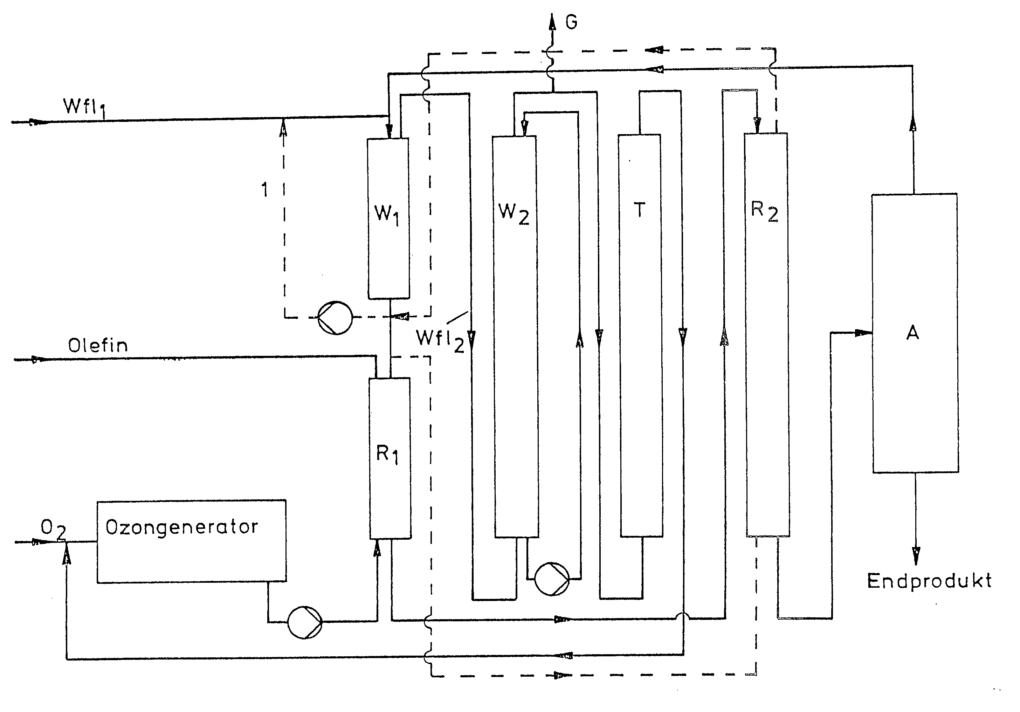

Das erfindungsgemässe Verfahren soll anhand der beigefügten Abbildung näher erläutert werden.

In einer ersten Reaktionsstufe ($R_1$) wird ein Gemisch von Olefin und Carbonsäure mit dem ozonhaltigen Gasstrom in Kontakt gebracht. Der Reaktor kann z.B. als Rührkessel mit einer in die Flüssigkeit eintauchenden Gaseinleitung ausgelegt sein. Als eine Anordnung, die sowohl vollständigen Olefin- als auch vollständigen Ozonumsatz ermöglicht, hat sich z.B. ein Blasen- bzw. Rieselsäulenreaktor, in dem Gas (von unten) und Flüssigkeit (von oben) im Gegenstrom geführt werden, als besonders vorteilhaft erwiesen.

In der ersten Reinigungs- oder Waschstufe ($W_1$) werden sodann die den Ozonolysereaktor verlassenden ozonfreien oder doch zumindest weitgehend ozonfreien Abgase mit der als Lösemittel für das Olefin verwendeten Carbonsäure mit einem Carbonsäureanhydrid oder einem Carbonsäure-Carbonsäureanhydrid-Gemisch gewaschen (Waschflüssigkeit Wfl. 1).

Der Ablauf der 1. Reinigungsstufe ($W_1$) wird dem Ozonolysereaktor zugeführt. Die Reinigungsstufe $W_1$ kann z.B. so ausgelegt sein, dass man in einer Rieselsäule von unten das zu reinigende Gas der Waschflüssigkeit Wfl. 1 entgegenführt. Reicht die als Lösemittel für die Ozonolyse benötigte Zulaufmenge an Carbonsäure aus, um bei einfachem Durchsatz durch die Waschsäule eine effektive Gasreinigung zu gewährleisten, kann auf einen Kreislauf (1) der Waschflüssigkeit Wfl. 1 verzichtet werden. Ist zur Erhöhung des Flüssigkeitsdurchsatzes der 1. Reinigungsstufe ($W_1$) ein Kreislauf nötig, so wird der Zulauf zum Waschkreislauf so bemessen, dass die Lösungsmittelentnahme für die Ozonolysereaktion sowie die Verluste durch Austragen mit dem Gasstrom kompensiert werden.

Das die 1. Reinigungsstufe ($W_1$) verlassende Abgas wird zweckmässigerweise, um die Verluste an Waschmedien mit hohem Dampfdruck so gering wie möglich zu halten, vor einer weiteren Wäsche durch Kondensation teilweise von seinen organischen Inhaltsstoffen befreit. Man kann dabei z.B. so vorgehen, dass man das Gas auf eine Temperatur abkühlt, die oberhalb des Erstarrungspunktes des in der Stufe $W_1$ verwendeten Waschmediums liegt, und die organischen Inhaltsstoffe als flüssiges Kondensat teilweise zurückgewinnt.

Der gegebenenfalls so vorbehandelte Gasstrom gelangt nun in die 2. Reinigungsstufe ($W_2$) und wird hier von dem aus der 1. Stufe stammenden Lösemittel bzw. Resten desselben befreit. Als Waschflüssigkeit (Wfl. 2) werden mindestens 0,1 gewichtsprozentige wässrige Lösungen alkalisch reagierender Substanzen, wie z.B. Hydroxide, Carbonate und Hydrogencarbonate von Alkali- bzw. Erdalkalimetallen, verwendet. Typische Vertreter sind NaOH, KOH, $Na_2CO_3$ und $HaHCO_3$. Sofern kohlendioxidhaltige Gasmischungen für die Ozonerzeugung verwendet werden, ist die Verwendung von Hydrogencarbonatlösungen als Waschmedium sinnvoll.

Die Wäsche in der 2. Reinigungsstufe ($W_2$) kann z.B. so durchgeführt werden, dass man in einer Rieselsäule, Blasensäule oder Glockenbodenkolonne von unten das zu reinigende Gas der im Kreis gepumpten Waschflüssigkeit entgegenführt.

Das die 2. Reinigungsstufe ($W_2$) verlassende Abgas wird zweckmässigerweise, um die Belastung der nachgeschalteten Trocknung so gering wie möglich zu halten, durch Kondensation soweit wie möglich von mitgeführtem Wasserdampf befreit. Auch hier kann wie bei dem der 1. Reinigungsstufe ($W_1$) nachgeschalteten Kühlsystem verfahren werden.

In der 3. Reinigungsstufe (T) wird das Abgas schliesslich durch geeignete Trocknungsverfahren bis auf einen Taupunkt $\leq -20\,°C$, vorzugsweise jedoch $\leq -50\,°C$, von Feuchtigkeit befreit. Das Trockenmittel kann in Türmen angeordnet sein und darin vom zu trocknenden Gas durchströmt werden. Als Trockenmittel können z.B. $CaCl_2$, $NaSO_4$, $P_2O_5$ und Silicagel eingesetzt werden. Bei einem technischen Verfahren setzt man vorzugsweise ein Molekularsieb geeigneter Porengrösse ein, da dieses die Möglichkeit einfacher Regeneration bietet. Um einen kontinuierlichen Betrieb der Ozonolyseanlage zu gewährleisten, ist es zweckmässig, 2 parallelgeschaltete Trocknungseinheiten wechselweise zu betreiben.

Das von organischen Inhaltsstoffen und Feuchtigkeit befreite Gas kann nun in den Ozongenerator handelsüblicher Bauart zur erneuten Ozonerzeugung eingesetzt werden. Zur Aufrechterhaltung des Gaskreislaufs und Überwindung des in der Apparatur sich aufbauenden Gegendrucks wird das Gas mit einem geeigneten Kompressor oder Gebläse verdichtet. Um den Pegel an nichtorganischen Bestandteilen (wie z.B. $N_3$, Ar, $CO_2$ etc.) möglichst niedrig zu halten, wird zweckmässigerweise ständig ein Seitenstrom aus dem Gaskreislauf ausgeschleust (Gasausschleusung G). Zum Ersatz dieser Gasverluste sowie des zur Ozonerzeugung verbrauchten Sauerstoffs wird ständig Frischgas eingespeist.

Das die Ozonolysestufe verlassende flüssige Reaktionsgemisch wird im allgemeinen in einer Nachbehandlungsstufe ($R_2$) entweder reduktiv, thermolytisch oder oxidativ-thermolytisch in stabile Endprodukte überführt. Nach beendeter Nachbehandlung wird das Reaktionsgemisch nach bekannten Verfahren aufgearbeitet und das hierbei zurückgewonnene Lösungsmittel als Waschmedium in die erste Reinigungsstufe ($W_1$) zurückgeführt.

Für den Fall einer oxidativ-thermolytischen Nachbehandlung sind z.B. folgende Verfahrensvarianten möglich:

(1) Das Ozonolysegemisch wird bei erhöhter Temperatur, die je nach Aufgabenstellung bis zu 150 °C betragen kann, mit einem sauerstoffhaltigen Gasgemisch oxidiert, das eine andere Zusammensetzung als das zur Ozonerzeugung benutzte Einsatzgas bzw. das Abgas der Ozonolysestufe hat. In diesem Falle kann für die Nachbehandlung ein eigener Gaskreislauf eventuell mit geeigneten Reinigungsstufen installiert werden. Handelt es sich bei dem zur Nachoxidation benutzten Gas um Luft, so kann auf eine Rückführung des Gases verzichtet werden.

(2) Das Ozonolysegemisch wird wieder bei erhöhter Temperatur mit einem Teil des die Ozonolysestufe verlassenden Abgases oxidativ nachbehandelt. Das die Nachbehandlung verlassende Abgas wird nach der Kondensation der organischen Inhaltsstoffe dem Gaskreislauf der Ozonolyse vor der 1. Reinigungsstufe ($W_1$) wieder zugeführt.

Zum Ersatz des in der Nachbehandlung verbrauchten Sauerstoffs muss dann eine erhöhte Menge an Frischgas in den Gaskreislauf der Ozonolyse eingespeist werden. Gelingt es, den Sauerstoff des zur Nachbehandlung eingesetzten Gases weitgehend oder völlig umzusetzen, so entfällt eine Einspeisung des Abgases der Nachbehandlung in den Gaskreislauf der Ozonolyse. Auf die Ausschleusung eines Seitenstroms aus dem Gaskreislauf der Ozonolyse zur Entfernung anorganischer Bestandteile ($N_2$, Ar, $CO_2$ etc.) kann in einem solchen Fall verzichtet werden.

Die gewerbliche Anwendbarkeit des erfindungsgemässen Verfahrens ist bei allen Ozonolyseprozessen, die in carbonsaurem Medium durchgeführt werden, gegeben.

Alle Prozentangaben sind, sofern nicht anders angegeben, Gewichtsprozente.

Das nachfolgende Beispiel dient zur Erläuterung des beanspruchten Verfahrens.

Beispiel

Der Ozonolysereaktor wird pro Stunde mit 166 Gewichtsteilen Cyclododecen beschickt. Über die 1. Reinigungsstufe hat der Ozonolysereaktor ferner einen Zulauf von 830 Gewichtsteilen Propionsäure pro Stunde. Den flüssigen Komponenten des Reaktionsgemisches wird in dem als Rieselsäule ausgebildeten Ozonolysereaktor, der durch Wasser mit einer Temperatur von 20 °C gekühlt wird, von unten ein 2,41 Volumenprozent Ozon enthaltender Sauerstoffstrom mit 48 Gewichtsteilen Ozon pro Stunde entgegengeführt. Die sich durch eine Temperaturerhöhung im Reaktorbett um ca. 20 °C bemerkbar machende Reaktion wird so geregelt, dass die Reaktionszone sich stets etwa in der Mitte des Ozonolysereaktors befindet. Auf diese Weise ist vollständiger Olefin- und Ozonumsatz gewährleistet. Das flüssige Reaktionsgemisch fliesst kontinuierlich ab und wird einer oxidativ-thermolytischen Nachbehandlung zugeführt.

Der den Ozonolysereaktor verlassende nunmehr ozonfreie Sauerstoff wird in einer Rieselsäule mit 830 Gewichtsteilen Propionsäure pro Stunde gewaschen. Die Verweilzeit der Propionsäure in der Rieselsäule beträgt bei einer Querschnittsbelastung von 66,4 g Propionsäure/cm² · h 2,5 Stunden. Der Gehalt an $C_{12}$-Verbindungen sowie kürzerkettigen Abbauprodukten im Abgas der Reinigungsstufe liegt zusammen unter 1 ppm.

Nach der 1. Reinigungsstufe wird der Gasstrom durch Kondensation bei einer Kühltemperatur von − 18 °C soweit von Propionsäure befreit, dass pro Stunde nur noch ca. 3 Gewichtsteile Propionsäure ausgetragen werden, die durch anschliessende Gegenstromwäsche mit 10%iger Natronlauge entfernt werden. Beim Umpumpen einer Natronlaugemenge von 60 000 Gewichtsteile pro Stunde und Einhalten einer Verweilzeit von 15 Minuten sowie einer Querschnittsbelastung von 1 200 g Natronlauge/cm² · h erreicht man, dass die Propionsäure im Abgas auf Werte unter 0,5 ppm ausgewaschen wird.

Das so gereinigte Gas wird durch Solekühlung (+ 5 °C) vorgetrocknet und anschliessend in einer Trockensäule, die mit einem handelsüblichen Molsieb gefüllt ist, bis auf einen Taupunkt < − 50 °C von Feuchtigkeit befreit. Die Belastung der Trockensäule beträgt 61 l Gas/l Trockenmittel · h.

Nach Zuführung des durch Reaktion verbrauchten bzw. Ausschleusung verlorenen Sauerstoffs durch Frischgas mit einem $O_2$-Gehalt > 99,5 Volumenprozent wird das Gas erneut zur Ozonerzeugung in einem nach dem Siemens'schen Röhrenprinzip arbeitenden Ozongenerator eingesetzt. Nach Verlassen des Ozongenerators wird der ozonhaltige Sauerstoff mittels eines Gebläses zur Überwindung des in der Anlage anstehenden Gegendrucks verdichtet.

Das Ozonolysegemisch wird nach Zusatz von 200 Gewichtsteilen Wasser in einem als Blasensäule ausgebildeten Reaktor oxidativ-thermolytisch nachbehandelt. Die Verweilzeit des Einsatzgemisches beträgt 10 Stunden bei einer Querschnittsbelastung von 24 g Flüssigkeit/cm² · h. Durch 3 getrennte Heiz- bzw. Kühlzonen werden 3 unterschiedliche Temperaturniveaus im Blasenreaktor eingestellt: oberes Drittel 70 °C, mittleres Drittel 90 °C, unteres Drittel 100 °C. Dem von oben zugeführten Einsatzgemisch werden 5% des Abgases der Ozonolysestufe als Oxidationsmittel entgegengeführt. Das die Blasensäule verlassende Abgas wird nach weitgehender Kondensation der organischen Bestandteile dem Gaskreislauf der Ozonolyse vor der 1. Reinigungsstufe wieder zugeführt.

Das den Nachreaktor verlassende Reaktionsgemisch wird abgekühlt. Das hierbei ausfallende Rohprodukt wird abfiltriert. Der nach Einengen des Filtrats erhaltene Rückstand wird mit dem Rohprodukt vereinigt und in Propionsäure umkristallisiert. Man erhält 83% Ausbeute Dodecandisäure in einer Reinheit von 99%.

**Patentansprüche:**

1. Verfahren zur Umsetzung von Olefinen in carbonsaurem Medium mit Ozon unter Verwendung von reinem Sauerstoff bzw. einer sauerstoffhaltigen Gasmischung zur Ozonerzeugung, dadurch gekennzeichnet, dass man den die Ozonolysestufe verlassenden Sauerstoff bzw. die sauerstoffhaltige Gasmischung

a) mit der in der Ozonolysestufe eingesetzten Carbonsäure wäscht, sodann

b) mit einer wässrigen Lösung einer alkalisch reagierenden Substanz behandelt, schliesslich

c) trocknet und in den Ozongenerator zurückführt.

2. Verfahren nach Anspruch 1, dadurch gekennzeichnet, dass man nach der ersten und/oder zweiten Verfahrensstufe eine Kondensation durchführt.

3. Verfahren nach den Ansprüchen 1 und 2, dadurch gekennzeichnet, dass man einen Teil des die Ozonolysestufe verlassenden Gases zu einer oxidativen Nachbehandlung des Reaktionsproduktes der Ozonolysestufe verwendet.

4. Verfahren nach Anspruch 3, dadurch gekennzeichnet, dass man das die oxidative Nachbehandlung verlassende Restgas dem Gaskreislauf des Ozonolyseverfahrens nach der Ozonolysestufe wieder zumischt.

**Revendications**

1. Procédé de réaction d'oléfines sur l'ozone dans un milieu d'acide carboxylique avec utilisation d'oxygène pur ou d'un mélange gazeux contenant de l'oxygène pour la préparation de l'ozone, caractérisé par le fait que:

a) On lave, avec l'acide carboxylique utilisé à l'étape d'ozonolyse l'oxygène ou le mélange gazeux contenant de l'oxygène qui quitte l'étape d'ozonolyse,

b) On le traite par une solution aqueuse d'une substance à réaction alcaline et enfin,

c) On le sèche et on le ramène au générateur d'ozone.

2. Procédé selon la revendication 1, caractérisé par le fait que l'on conduit une condensation après la première et/ou la deuxième étape du procédé.

3. Procédé selon les revendications 1 et 2, caractérisé par le fait que l'on utilise une partie du gaz quittant l'étape d'ozonolyse pour un posttraitement d'oxydation du produit de réaction de l'étape d'ozonolyse.

4. Procédé selon la revendication 3, caractérisé par le fait que l'on incorpore à nouveau le gaz résiduel quittant le posttraitement d'oxydation au circuit de gaz du procédé d'ozonolyse, après l'étape d'ozonolyse.

**Claims**

1. A process for the reaction of an olefin in a carboxylic acid medium with ozone, pure oxygen or an oxygen-containing gas mixture being used for the production of the ozone, characterised in that the oxygen or oxygen-containing gas mixture leaving the ozonolysis step is

a) washed with the carboxylic acid charged to the ozonolysis stage, then

b) treated with an aqueous solution of an alkaline-reacting material, and subsequently

c) dried and returned to the ozone generator.

2. A process according to claim 1, characterised in that a condensation is carried out after the first and/or after the second of these process steps.

3. A process according to claims 1 and 2, characterised in that a portion of the gas leaving the ozonolyses stage is used for an oxidative after-treatement of the reaction product of the ozonolysis stage.

4. A process according to claim 3, characterised in that the residual gas leaving the oxidative after-treatement is remixed into the gas circulation system of the ozonolysis stage.

- 1/i

A

Endprodukt

R 2

T

W 2

G

Wfl 2

W 1

R 1

Wfl 1

Olefin

Ozongenerator

O 2

7

1